# EUROPEAN PATENT SPECIFICATION

(11) **EP 4 154 922 B1**
(45) Date of publication and mention of the grant of the patent: **27.05.2026**
(21) Application number: 21739777.7
(22) Date of filing: 18.05.2021
(51) Int. Cl.: A61L 15/28, A61L 15/46

(54) **WOUND TREATMENT DRESSING, METHOD OF PRODUCING SAME AND USES THEREOF**
WUNDVERBAND, VERFAHREN ZU SEINER HERSTELLUNG UND DESSEN VERWENDUNG
PANSEMENT, PROCEDE DE FABRICATION ET D'UTILISATION

(30) Priority: 18.05.2020 PT 2020116388
(43) Date of publication of application: 29.03.2023
(73) Proprietor: Falcon Empire Lda., 6000-767 Castelo Branco (PT)
(72) Inventor: NUNES AGOSTINHO, Filipe José, 6000-473 Castelo Branco (PT); GARCIA FRADE, Paulo Jorge, 2695-166 Santa Iria De Azoia (PT)
(74) Representative: Patentree
(86) International application number: PCT/IB2021/054271
(87) International publication number: WO 2021/234568

(56) References cited:
- EP-A2- 1 064 956
- EP-A2- 1 654 114
- EP-B1- 1 654 114
- CN-A- 101 669 967
- KR-A- 20120 026 324
- KR-A- 20200 122 527
- US-A1- 2005 037 680
- US-A1- 2015 190 290
- US-B1- 6 348 423
- US-B2- 9 427 363
- YUWONO HENDRO SUDJONO: "The New Paradigm of Wound Management Using Coffee Powder", GLOBAL JOURNAL OF SURGERY, vol. 2, 1 January 2014 (2014-01-01), pages 25 - 29, XP055835285, Retrieved from the Internet <URL:http://www.sciepub.com/portal/downloads?doi=10.12691/js-2-2-2&filename=js-2-2-2.pdf> DOI: 10.12691/js-2-2-2

## Description

### TECHNICAL FIELD

The present disclosure concerns a wound treatment dressing, in particular to prevent/eliminate odours, which comprises a textile material with the incorporation of coffee grounds to neutralize odours from exudates from chronic wounds. The selection of coffee grounds as a functional agent to be incorporated into the textile was related to its odour neutralizing properties, as well as its potential valorisation as a waste.

Namely, a wound treatment dressing, in particular for the absorption of odours in wounds, including pressure ulcers, leg ulcers, cancerous wounds, diabetic foot ulcers, burns, traumatic or surgical wounds, which comprises an odour-inhibiting mixture with coffee grounds.

### BACKGROUND

Acute and chronic wounds affect about 2% of the population, representing close to 4% of total health care expenditure and approximately 68% of nurses' working time. Of all the symptoms of the wound, the odour is referred by patients and caregivers as the most pungent, causing social isolation, depression, and repulsion.

The bad odour arises from a combination of factors, including bacteria, necrotic tissue, high levels of exudate and poorly vascularized tissue. It is caused by a mixture of volatile compounds, which includes short-chain organic acids (n-butyric, n-valeric, n-caproic, n-heptanoic and n-caprylic), produced by anaerobic bacteria, together with a mixture of amines and diamines, such as cadaverine and putrescine, which are produced in the metabolic processes of other proteolytic bacteria. This odour has been linked to the smell of rotting meat. Recently, dimethyltrisulfide has also been identified in chronic wounds as a source of odour. This compound was found in volatiles released from certain vegetables, fermented milk and aged foods or drinks, being produced by aerobic bacteria such as *Pseudomonas aeruginosa.*

The smell is difficult to identify, as any odour can be made up of hundreds of different chemicals. It was believed that humans could adapt to any odour, however in the presence of putrescine and cadaverine they tend to feel like coughing, due to the high toxicity of these compounds.

Recently, Gethin *et al.* evaluated the performance and effectiveness of some topical wound agents, as well as some odour control compounds, present in the environment to which patients are exposed. With this study, they found that activated carbon dressings are the most used, as they are more effective in eliminating bad odour from the wound (about 48.8%), while coffee and coal are two of the products referred by users as potential odour neutralizers.

Coffee is one of the most popular and appreciated drinks worldwide, being consumed for its stimulating and refreshing properties, which are defined by the composition of the coffee tree and the changes that occur during the roasting process. As a result of this large market, the coffee sector is responsible for producing large quantities of waste - spent coffee grounds (SCG) and coffee silverskin (CS). SCGs correspond to the powder that remains after the extraction of the drink - grounds, being a resource found globally, which is given little importance.

SCG represent the most abundant and non-edible agricultural waste obtained by the wet processing of red berries of coffee. Caffeine and chlorogenic acid derivatives (CGA) are found not only in coffee but also in wasted residues, even presenting therapeutic properties. CGA, especially, are of great importance to humans due to their antioxidant properties [L. F. B. Giraldo, "Extraction and characterization of polysaccharides and phenolic compounds from spent coffee grounds and their incorporation into edible films/coatings for food applications", 2016].

Coffee residues, in addition to those mentioned, contain large amounts of organic compounds (fatty acids, lignin, cellulose, hemicellulose and other polysaccharides) that justify the valorisation thereof. Some researchers have already analysed coffee waste as a biological resource for various purposes, such as for the production of biodiesel, as a source of sugars, as a precursor to the production of activated carbon, as a compound and as an adsorbent for the removal of metal ions.

The removal of pollutants from heavy metals, such as lead, for example, has been of great research interest due to the significant risks of the pollutants to human health and the environment. Of the various existing techniques, adsorption was considered the most practical, inexpensive, and efficient method for reducing lead and other heavy metal pollutants. Ayucitra *et al.* evaluated and used various residues as precursors of bioadsorbents in an attempt to eliminate contaminants from wastewater. Roasted coffee was analysed as a potential alternative material for bioadsorption due to its high carbon content (around 50.6%).

Gizaw *et al.* found that SCG are made up of a complex community of non-*Saccharomyces* yeasts. As such, they are a good substrate to house other yeast species, which indicates that they provide a favorable environment for their development and proliferation. In this sense, it is not advantageous that SCGs are directly in contact with the exudate of chronic wounds, as they can cause an aggravation of the wound. It is suggested the insertion of an antimicrobial agent or the sterilization of SCG to eliminate this problem.

It is necessary to continue exploring the application of SCG and CS, taking into account their profitable use, adding the value of these unused materials and reducing their impact on the environment. Although some characteristics of these compounds have already been reported recently in the literature, there is no study that presents a complete characterization of the two materials.

Wound care has evolved since ancient times. Initially, the application of curative materials aimed at inhibiting bleeding and protecting the wound from environmental irritation, as well as water and electrolyte disturbances. The skin plays an important role in homeostasis and in preventing invasion by microorganisms and as such it needs to be covered much of the time with a bandage after the injury. There are three categories of dressings: biological, synthetic, and biological-synthetic. In the case of biologicals, some of them have disadvantages, such as high antigenicity, low adhesion and risk of cross contamination. Synthetic dressings have a long lifespan, induce a minimal inflammatory reaction and have almost no risk of pathogen transmission. Bio-synthetic dressings have bilayers and are made of high density polymers and biological materials.

These three categories of dressings are all used frequently in clinical settings, but all of them have disadvantages.

An ideal dressing should maintain a moist environment at the wound interface and allow exchange of gases, acting as a barrier to microorganisms and removing the excess exudates. It should also not be toxic, allergenic, sticky and should be easily removed without trauma. In addition, it should be made based on a biomaterial easily accessible, requiring minimal processing, having antimicrobial properties.

Various products such as gauze, hydrogels, foams, hydrocolloids (carboxymethylcellulose), alginate, chitosan, collagen, cellulose, cotton/rayon, transparent films (polyurethane) are recommended as passive dressings for wounds and burns, due to their actions in the local cell responses.

In recent years, a large number of research groups have devoted themselves to producing new and improved wound dressings, synthesizing, and modifying biocompatible materials. Current strategies are also focused on accelerating wound repair by systematically engineered healing materials. In particular, efforts are focused on the use of biologically derived materials, such as chitin and its derivatives, which are able to accelerate healing processes at molecular, cellular and systemic levels.

Chitin is an easily available and inexpensive biological material, obtained from the skeleton of invertebrates, as well as from the cell wall of fungi. It is a copolymer of units of N-acetyl-D-glucosamine linked by glycoside linkage (1-4), wherein the units of N-acetylglucosamine are predominant in the polymer chain. Chitin and its derivatives, such as chitosan, are biocompatible, biodegradable, non-toxic, antimicrobial and moisturizing. Due to these properties, they have good biocompatibility and positive effects on wound healing. However, the low solubility of chitin causes some limitations in its applications.

These biopolymers have been studied in several applications as a raw material not only for absorbable sutures, wound dressings and synthetic fibers, but also in the environmental area in the treatment of effluents. Previous studies show that chitin-based dressings can accelerate the repair of different tissues, facilitating the contraction of wounds and regulating the secretion of inflammatory mediators. Chitosan demonstrated a good effect on the wound healing/cicatrisation process. Chitin can also be used as a coating on normal biomedical materials [S. Pokhrel, P.N. Yadav, and R. Adhikari, "Applications of chitin and chitosan in industry and medical science: A review", Nepal J. Sci. Technol., Vol. 16, no. 1, pp. 99-104, 2015].
Another problem, found in current commercial dressings, is the fact that it is not as effective in the most peripheral areas. In this sense, the treatment of wounds using ground coffee has been recognized as an alternative medicine, considering its effectiveness in wound healing. *Yuwono* (2014), after conducting a study in this area, stated that the use of coffee powder as a wound dressing can have a strong influence on the emergence of a new paradigm for the treatment of wounds. This is because the coffee powder promotes a more prolonged action since it easily absorbs the fluid from the wound and can confer antioxidant activity and antibacterial properties to it. Thus, wound healing is more rapid and prevents the risk of contamination and microbial infection. In addition, it allows the absorption and neutralization of odour, minimizes pain, is more cost-effective and does not cause adverse reactions. In terms of the deodorant action, coffee as it is known has a characteristic fragrance, so it is very useful to mask the odour of wounds. These results provide confidence for scientific research in the use of coffee grounds, safely, in a wound treatment dressing [H. S. Yuwono, "The New Paradigm of Wound Management Using Coffee Powder", Glob. j. Surg., Vol. 2, no. 2, pp. 25-29, 2014].

Many health professionals from areas of coffee plantations have also reported that coffee powder on acute or chronic wounds (diabetes mellitus, sharp cuts, burns) does not cause infections. That is, the coffee powder can come in contact with the wound until its healing. Perry *et al.* tested the effect of coffee grounds from East and Southeast Asia and state that they can be used to treat burns.

Document BR10201202916 discloses a composition and a formulation thereof for topical use of coffee oil base with high capacity for wound healing. The composition presented is able to keep the scar area with ideal humidity and dry and protected outlying areas, in addition to presenting a systemic action, factors that accelerate the healing process.

In 2014, Hung *et al.* developed fibers with coffee residues, comprising a polymeric granulate, in order to combat unpleasant human odours released by different sources (biological decomposition, chemical agents, tobacco smoke, etc.) to the environment. The production of this yarn included the compression of a material based on carbonized particles from ground coffee beans and coffee grounds, without organic content. The types of polymers used varied between polyester, nylon or polyethylene terephthalate in fabric, non-woven fabric or knitwear. This invention is intellectual property of the S.Café^{®} brand, its application being essentially used in sports clothing with the intrinsic properties of quick drying, sweat odour control and moisture reduction.

In turn, also in 2014, Carlucci *et al.* created absorbent personal hygiene articles to reduce related bad odours, acting directly on the released compounds or indirectly on the users' olfactory system. As hygiene items they included sanitary towels, daily pads, adult incontinence articles, baby diapers, paper towels, bath tissues and facial tissues that are used to absorb and retain body fluids and other exudates excreted by the human body. The term "absorbent article" has been used in a very broad sense including any article capable of receiving and/or absorbing and/or containing and/or retaining fluids and/or exudates, especially body fluids/body exudates. In addition, these articles were planned to be disposable, that is, for single use and in order to be compatible with the environment. There are numerous substances that have the capacity to absorb/adsorb and neutralize unpleasant odours, including zeolites, starch, activated carbon, cyclodextrins, chitin or chitosan. In addition, there are functional groups such as esters and aldehydes with this functionality, wherein in this case the aldehydes proved to be more useful ones.

The Carboflex^{®} commercial dressing, marketed by Convatec^{®}, is a sterile, non-adhesive dressing designed for the management of malodorous wounds. It has five layers, one of which consisting of activated carbon, with a high odour retention surface.

The Actisorb Silver 220^{®} commercial dressing, marketed by Systagenix^{®}, consists of an activated carbon knitted fabric, impregnated with silver, which in turn is wrapped in a non-woven nylon.

Document EP1654114A2 discloses wound care devices having a topically applied silver-based antimicrobial finish are provided. The finish comprises at least one silver ion-containing compound and at least one binder compound. The finish may be applied to a target substrate, such as a fiber, fabric, film, foam, hydrogel, or hydrocolloid to provide a single layer antimicrobial wound care device. Alternatively, a silver-containing layer may be combined with one or more additional layers of target substrate to provide a composite antimicrobial wound care device. The device may also contain an odor-absorbing component capable of reducing or eliminating odors that are inherently associated with infectious wounds. Also provided is a method for making the wound care device and a composition of matter comprising the silver-based antimicrobial finish.

These facts are described in order to illustrate the technical problem solved by the embodiments of this document.

### GENERAL DESCRIPTION

The present disclosure relates to a wound treatment dressing, with incorporation of spent coffee grounds for odour neutralization, preferably derived from chronic wound exudates although capable of being used in any malodorous wound. The selection of spent coffee grounds, as a functional agent to be incorporated into the textile, was related to its odour masking properties, as well as its potential valorisation as a waste.

Therefore, one of the main objectives of this disclosure was to assess the potential for incorporating spent coffee grounds into a textile material (woven or non-woven fabric) as a functional agent for neutralizing odours from wound exudates.

In an initial phase, the selected material was characterized - spent coffee grounds, for incorporation into the textile material. After the characterization of the functional agent, it was incorporated into several textile substrates and their respective characterization in terms of odour adsorption/neutralization capacity, using gas chromatography with flame ionization detection (GC-FID). At the same time, a sensory evaluation panel was used to assess the intensity of the odour released by the developed dressings. In addition to these tests, the rate of dehydration and the absorption capacity of the dressings were determined, using an ionic solution that simulates wound exudate. Regarding the skin contact that this disclosure may have, the loss of transepidermal water and skin erythema was evaluated through corneometry tests.

Throughout the development of this disclosure, the most common commercial solutions for this purpose, based on activated carbon, were tested, always establishing a comparison with the results obtained with this disclosure.

A wound treatment dressing is described which comprises: an absorbent layer comprising an odour-inhibiting mixture; wherein the odour-inhibiting mixture comprises spent coffee grounds as defined in the claims.

The granulometry of spent coffee grounds varies between 3-5000 µm; preferably between 4-4000 µm; more preferably 5-3000 µm and the coffee grounds are dried and/or ground.

The measurement of granulometry/particle size/coffee grounds granule can be performed in several ways, in this disclosure the measurement of granulometry/particle size was carried out through the Leica DM 2500M optical microscope, using the *Leica Application* Suite software.

The odour-inhibiting mixture comprises **20** - 25% (w/V inhibiting mixture) of spent coffee grounds

In an embodiment, the absorbent layer comprises between 0.05 to 5 g of spent coffee grounds per cm² of absorbent layer; preferably between 0.02 to 2.5 g of spent coffee grounds per cm² of absorbent layer.

In a preferred embodiment, the dressing further comprises: an outer layer comprising a textile substrate, which serves as a support for the absorbent layer; an absorbent layer comprising a textile substrate, which serves as a support for the inhibiting mixture.

The odour-inhibiting mixture comprises an adhesion agent in which the odour-inhibiting mixture comprises 0.5-5% (V/V_{inhibiting mixture}) of the adhesion agent; and 20-25% (w/V_{inhibiting mixture}) of spent coffee grounds.

The odour-inhibiting mixture further comprises a dispersant, a binding agent, and a buffer solution.

In an embodiment, the absorbent layer further includes an active ingredient, a moisturizer, a disinfectant, an anesthetic agent or combinations thereof.

In an embodiment, the absorbent layer comprises between 0.05 to 5 g of spent coffee grounds per cm² of absorbent layer.

In an embodiment, the absorbent layer is coated using a Meyer bar and/or film applicator wherein the wire diameter used in Meyer bar rod comprises between 100-110 µM.

In an embodiment, the dressing further comprises an outer layer that comprises a textile substrate, which serves as a support for the absorbent layer carrying the inhibiting mixture.

In an embodiment, the absorbent layer further comprises superabsorbent polymers.

In an embodiment, the adhesion agent is selected from a list comprised of: a highly active matrix based on anionic, cationic, nonionic, amphoteric agents or combinations thereof, preferably based on siloxanes.

In an embodiment, the binding agent is selected from a list comprised of: chitosan, gelatin, pectin, cellulose, cellulose derivatives, glucomannan, acrylic emulsions, styrene-butadiene emulsions, styrene-acrylic emulsions, polyurethane-based dispersions, or combinations thereof.

In an embodiment, the dispersant is selected from a list comprised of: polyester, siloxane, polyphosphates, polyacrylates, or combinations thereof, preferably a copolymer of polyester and siloxane.

In an embodiment, the textile substrate is a natural fiber, a synthetic fiber or combinations thereof.

In an embodiment, the pH range of the buffer solution is comprised between 6 and 10.

In an embodiment, the textile substrate comprises cotton (CO), polyester (PES), non-woven fabric (TNT), or combinations thereof.

In an embodiment, the textile substrate comprises 100% CO or 63% CO/37% PES and TNT PES.

Also disclosed but not part of the invention, the present application relates to a composition comprising coffee grounds for the treatment of wounds; in particular exposed wounds, more particularly for odour control; wherein the composition comprising coffee grounds is administered in a transdermal dressing comprising: an absorbent layer comprising an odour-inhibiting mixture as defined in the claims.

Also described is the use of the dressing described in any of the previous embodiments as a wound odour absorber.

Also described is a method for producing a wound dressing as defined in claim 13 which comprises the following steps: preparing the odour-inhibiting mixture with spent coffee grounds as defined in the claims; placing the odour-inhibiting mixture in the absorbent layer; applying the absorbent layer on a textile substrate; drying in the oven, preferably infrared (IR) at 100 °C, the substrate with the composition; pressing or laminating the structure in order to obtain a dressing, preferably pressing at 110 °C with a pressure of 0.6 MPa (6 bar) for 1 minute.

In an embodiment, the method for producing a dressing according to the previous embodiment in which the spent coffee grounds are ground at 400 rpm, for 30 minutes, whenever the inhibiting mixture is prepared and the textile is further functionalized.

In an embodiment, the preparation of the binding agent comprises the following steps: preparing a mixture of water or a phosphate buffer solution; adding chitosan and acetic acid under mechanical/magnetic stirring until the chitosan is well dissolved and the solution is homogeneous; adding the spent coffee grounds to the chitosan solution; well mixing using a mechanical stirrer; adding the silane and keeping stirring until the mixture is well homogeneous.

The present embodiments have several advantages, which are listed below.

### BRIEF DESCRIPTION OF THE FIGURES

For an easier understanding, figures are herein attached, which represent preferable embodiments which are not intended to limit the object of the present description.
**Figure 1** - Illustration of an embodiment of the results of the reduction capacity of isovaleric acid (IVA) by the different layers of the commercial dressings Carboflex^{®} and Actisorb^{®}.
**Figure 2** - Illustration of a preferred embodiment of the techniques used in the coating: (a) there is the film applicator, (b) the knife coating and (c) the Meyer bar is present.
**Figure 3** - Illustration of an embodiment of an intersection edge of two fitted square sides of the half cube.
**Figure 4** **-** Illustration of an embodiment of the results of the IVA reduction capacity by TNT PES functionalized with the various compounds analysed and the respective formulation of the present embodiment.
**Figure** 5 - Illustration of an embodiment of the results obtained from IVA reduction capacity by the 100% CO fabric functionalized with the various compounds analysed and the respective controls under study.
**Figure 6** - Illustration of an embodiment of results obtained from IVA reduction capacity by the 100% CO and CO/PES fabric functionalized with different amounts of grounds and respective controls under study.
**Figure 7** - Illustration of an embodiment of the results obtained from the IVA reduction capacity by the CO/PES fabric functionalized on one side and on both sides with the formulation of ground and unground coffee grounds and respective controls.
**Figure 8** - Illustration of an embodiment of the IVA reduction capacity by the 100% CO fabric functionalized with the formulation of grounds at different concentrations of silane and respective controls.
**Figure 9** - Illustration of an embodiment of the IVA reduction capacity by the CO/PES fabric functionalized with the formulation of grounds at different concentrations of silane and respective controls.
**Figure 10** - Illustration of an embodiment of the IVA reduction capacity by the prototypes of dressings developed with the outside in TNT, inner layer 100% CO or CO/PES with formulation and the respective complete dressings.
**Figure 11** **-** Illustration of an embodiment of the IVA reduction capacity by the prototypes of complete dressings developed with the outside in TNT and/or CO/PES and respective controls, on different days and by different operators.
**Figure 12** - Illustration of an embodiment of the IVA reduction capacity over 24 hours, by the Actisorb^{®} dressing and the two dressings developed.
**Figure 13** - Illustration of an embodiment of the granulometries of ground coffee grounds.
**Figure 14** - Illustration of an embodiment of the diameter of the wire used in the rod of the Meyer bar.

### Detailed description

The present disclosure relates to a wound treatment dressing, in particular for the absorption of odours in wounds, including pressure ulcers, leg ulcers, cancerous wounds, diabetic foot ulcers, burns, traumatic or surgical wounds, which comprises an odour-inhibiting mixture with coffee grounds.

The present disclosure describes a wound treatment dressing as defined in the claims comprising: an absorbent layer comprising an odour-inhibiting mixture; wherein the odour-inhibiting mixture comprises spent coffee grounds. The granulometry of ground coffee grounds varies between 3-5000 µm; preferably between 4-4000 µm; more preferably 5-3000 µm and the coffee grounds are dried and/or ground. The dressing further comprises an outer layer comprising a textile substrate, which serves as a support for the absorbent layer; an absorbent layer comprising a textile substrate, which serves as a support for the inhibiting mixture. The odour-inhibiting mixture comprises an adhesion agent, where the odour-inhibiting mixture comprises 0.5 - 5% (V/V_{inhibiting mixture}) of the adhesion agent; and 20 - 25% (w/V_{inhibiting mixture}) of spent coffee grounds. The odour-inhibiting mixture further comprises a dispersant, a binding agent and a buffer solution.

The present disclosure describes a wound treatment dressing comprising: an absorbent layer comprising an odour-inhibiting mixture; wherein the odour-inhibiting mixture comprises coffee grounds. The wound treatment dressing of the present disclosure comprises a granulometry of ground coffee grounds that ranges from 5-5000 µm and are dried/ground.

In an embodiment, the dressing further comprises: an absorbent layer comprising a textile substrate, which serves as a support for the inhibiting mixture. It also describes a method for producing the dressing according to claim 13 that comprises the following steps:
preparing the odour-inhibiting mixture with spent coffee grounds as defined in the claims; placing the odour-inhibiting mixture in the absorbent layer; applying the absorbent layer on a textile substrate; drying in the oven, preferably infrared at 100 °C, the substrate with the composition; pressing or laminating the structure in order to obtain a dressing, preferably pressing at 110 °C with a pressure of 6 bar for 1 minute.

In order to compare the performance of the present disclosure' dressings, two types of commercial dressings were analysed, both indicated for the treatment of exudates and the management of chronic wound odour. These commercial dressings feature a layer of activated carbon as an odour-absorbing substrate. One of the main objectives of this disclosure consisted in the replacement of activated carbon by coffee grounds, since these show a high absorption/neutralization area and, at the same time, allowing to add value to a by-product of the coffee industry.

In an embodiment, the commercial dressings analysed were Carboflex^{®} and Actisorb Silver 220^{®}, marketed by the international companies Convatec^{®} and Systagenix^{®}, respectively.

In an embodiment, in order to evaluate the odour retention capacity of the commercial dressings studied, the gas chromatography technique with flame ionization detection (Gas Chromatography - Flame Ionization Detector, GC-FID) was used according to a procedure adapted from ISO 17299-3:2014 - Determination of deodorant properties - Part 3: Gas chromatography method. The gas chromatograph (GC) with flame ionization detector used corresponds to the 2010 Plus model of the Shimadzu brand. The capillary column is from the brand Teknokroma, model Meta.X5, nonpolar, 50 m long, with an internal diameter of 0.20 mm and a film thickness of 0.33 µm. The chromatograph has a "split/splitless" type injector, set to a split ratio of 1:5. The results were obtained using the GC Solution version 2.4 software, specific from Shimadzu.

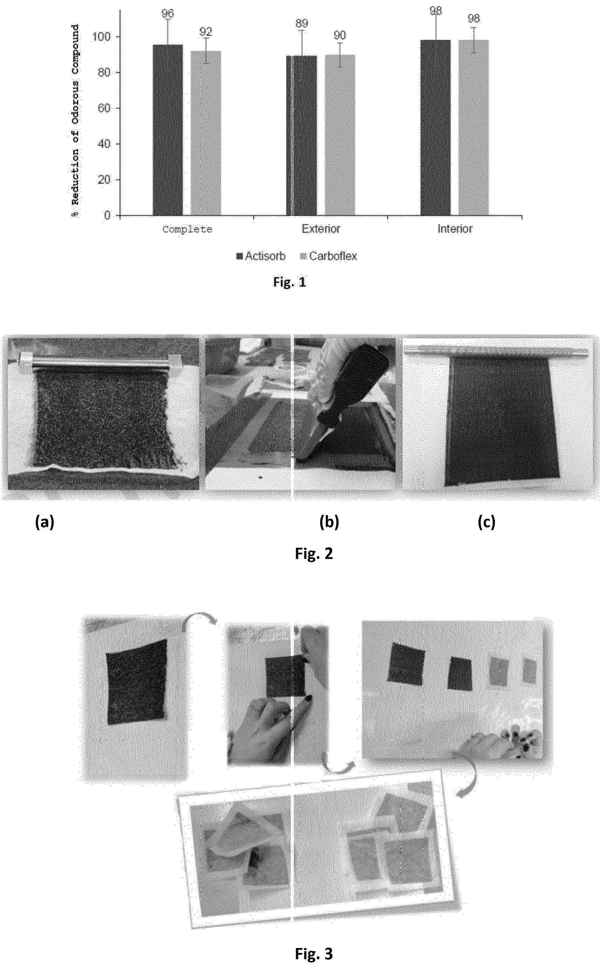

Commercial dressings, despite being considered efficient in terms of absorbing chronic wound exudates, after 24 hours in contact with the wounds have the problem of releasing the unpleasant odour of the exudate into the surrounding environment. Therefore, the IVA retention capacity of the different layers of the commercial dressings under analysis was evaluated, as shown in **Figure** 1. In the scope of this disclosure, only IVA was used, since it is one of the odorous compounds frequently found in the beds of patients with chronic wounds who, in turn, have bacterial colonization or infection.

In an embodiment, as shown in **Figure 1****,** it appears that commercial dressings show very similar results, in terms of the reduction of this odorous marker, by the different layers. Between layers it is also observed that there are no significant differences in the percentage of reduction in IVA, for both commercial dressings.

In an embodiment, taking into account that the minimum percentage of IVA reduction, referred to by the standard, for a fabric (dressing) to be considered deodorant regarding this odour, is 85%, it can be said that both dressings present this property. However, after each test, the textile layers were smelled and it was found that the outer layers of both dressings absorbed and retained the odour of the analysed odorous compound. This situation may mean that this layer acts as a barrier in the interaction of the compound with the inner layer (of activated carbon), promoting a lesser reduction of the odours released from the exudate of chronic wounds, when the dressing is complete. Regarding the inner layer, it was found that it had no smell after the test, which indicates that this layer of activated carbon has the capacity to neutralize the odour. The higher percentage of IVA reduction for the inner layer (≈ 98%), followed by the complete dressing (≈ 94%) and, in turn, the outer layer (≈ 90%) proves what has been stated above. In general, it is concluded that, in terms of the capacity to reduce odours, the two commercial dressings behave similarly.

In an embodiment, for the incorporation of spent coffee grounds into the textile substrate, the use of the Meyer bar, with a thickness of 100 µm, was used. This technique made it possible to easily obtain a uniform coating with the desired thickness, as shown in Figure 2. In this way, the Meyer bar was used in the incorporation of formulations with coffee grounds, in the different textile substrates. After the formulation was applied to the textile support, the coating was subjected to drying in an IR oven at 100 °C. Finally, the samples were pressed at 110 °C, with a pressure of 0.6 MPa (6 bar) for 1 minute.

In an embodiment, the textile material with spent coffee grounds was developed using the textile substrates of 100% (V/V) CO, 63% (V/V) CO/37% (V/V) PES and TNT PES.

In an embodiment, several formulations were evaluated to define the most suitable one either in terms of spreading, or in terms of the amount of grounds and their effect on the capacity to reduce odours.

In an embodiment, the addition of thickeners such as cellosize (hydroxymethylcellulose) and CMC (carboxymethylcellulose) to the coffee grounds was assessed, however the amounts tested made it difficult to spread the formulation.

In an embodiment, the presence of a binder such as chitosan, glucomannan (konjac fiber) and impranil was also assessed. Glucomannan, although promoting a good texture to the formulation, acts as a prebiotic agent stimulating the growth and/or activity of certain bacteria. In this sense, it is not at all advisable in medical terms, as the formulation may be in contact with a wound. Impranil is an acrylic polymer resin that creates a film on the textile support, preventing to some extent the absorption of odours by the coffee grounds. Regarding chitosan, its addition makes the formulation with a viscosity suitable for spreading and still provides antimicrobial properties, important conditions considering the application purpose. However, the solution of chitosan in acetic acid with the coffee grounds is not sufficient for the formulation to have a good adhesion and uniformity on the textile substrate.

In an embodiment, taking into account these problems, the addition of a dispersant, such as TEGO WET 240 (highly active compound based on siloxane), and an adhesion agent, such as silane, was evaluated. The dispersant mentioned, despite improving the texture of the formulation with the coffee grounds and its spreading, is a copolymer of polyester siloxane. As such, it is a very reactive compound, so it has a high odour reduction capacity. In this sense, its presence masks the effect of the grounds, which is not intended with the present disclosure. Regarding silane, its presence has made the adhesion of grounds to the textile more efficient. Although at concentrations between 5-10% (V/V) it has the same effect as TEGO, in terms of the capacity to reduce odours, at low concentrations (0.1-3% (V/V)) this is not found, not influencing the effect of coffee grounds.

In an embodiment, in terms of the capacity to reduce odours, grinding or not grinding did not show significant differences. However, the uniformity of the dispersion of the formulation applied to the textile improves considerably when using ground grounds. Therefore, it was decided to grind the coffee grounds in a ball mill at 400 rpm, for 30 minutes, whenever the textile was functionalized.

In an embodiment, the odour-inhibiting mixture with better results comprises 20-25% w/Vₜₒₜₐₗ spent coffee coffee grounds (SCG) in a 1% (w/V) solution of chitosan (Cs) in acetic acid (CH₃COOH 1% (V/V)) and 0.5% (V/V) silane. Due to the acidity of this formulation with a pH≈4, it was decided to bring the pH of the solution to 7, taking into account the possible contact with the skin and/or exudate from wounds. Thus, the amount of acetic acid (CH₃COOH) used was reduced to only 0.25% (V/V), using the phosphate buffer solution having pH≈7.4 in complementary manner.

In an embodiment, for the development of this embodiment, the inner layer of activated carbon of the commercial dressings was replaced by a coating with coffee grounds. In this sense, the present embodiment follows a structure similar to the dressings already existing on the market, being comprised by an inner layer and an outer layer.

In an embodiment, Figure 3 shows how to make an embodiment of the dressings of the present embodiment, using a thermally adhesive tape for their closure.

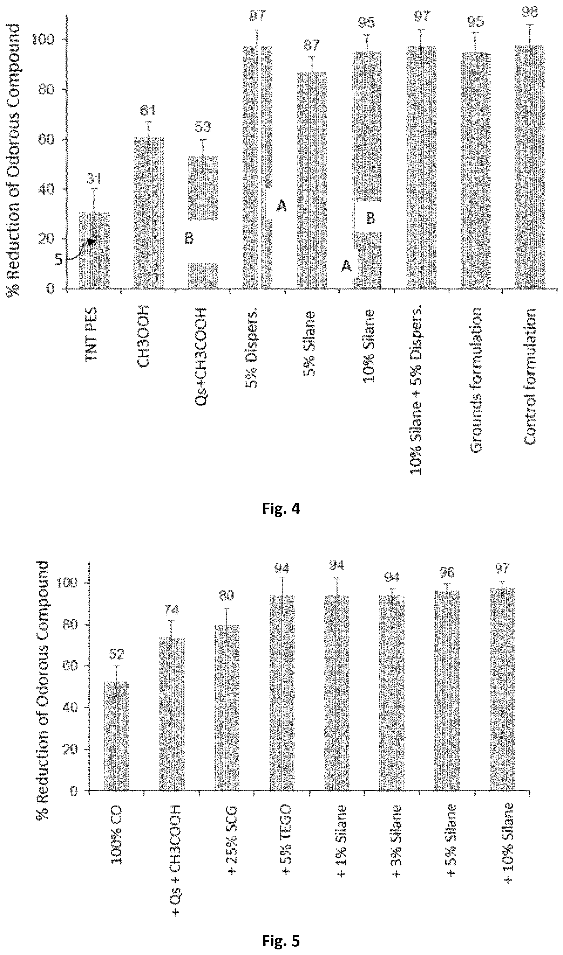

In an embodiment, through the analysis of Figure 4, it is possible to verify that both chitosan and acetic acid promote a small increase in the capacity of intrinsic reduction of IVA by TNT PES, although this is not significant. In contrast, 5% (V/V) of TEGO (dispersant) impregnated in the fabric promotes a considerable increase, which indicates that this compound masks the effect of coffee grounds when added together. The same occurs with 5% (V/V) and 10% (V/V) silane, although 5% (V/V) silane shows a lesser effect, which in a smaller percentage may be useful for binding the grounds to the textile and not masking the reduction capacity of the grounds themselves. These two compounds together provide a high IVA reduction capacity, taking into account their individual behaviors. The reduction capacity of the control fabric (everything but the grounds) and the fabric with the formulation had already been analysed and, in this case, it is present only for comparison.

In an embodiment, the results in Figure 5 suggest that the effect of both compounds, TEGO (dispersant) and silane, is the same on the different substrates analysed. It is further noted that when adding TEGO to the fabric with chitosan and grounds (SCG), the percentage of IVA reduction considerably increases. Thus, it is confirmed that TEGO masks the effect of grounds, as its addition immediately promotes a high IVA reduction capacity. Regarding silane, it was found that in the range of 1-10% (V/V), together with the other compounds, its IVA reduction effect is practically the same and quite high, which means that the formulation would already be saturated in terms of odour reduction capacity with the addition of TEGO (5% (V/V).

In an embodiment of the present embodiment, it was decided to apply a formulation with only spent coffee grounds in a 1% (w/V) solution of chitosan in acetic acid, in 100% (w/w) CO and 63% (w/w) CO / 37% (w/w) PES fabrics. In order to compensate for the removal of dispersant and silane in the formulation, it was decided to increase the amount of coffee grounds to 30% w/Vₜₒₜₐₗ.

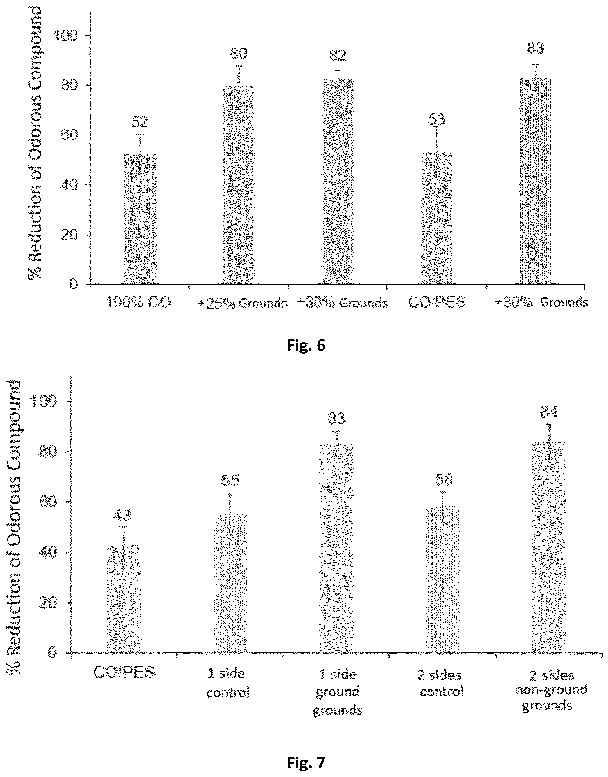

In an embodiment, the results of the chromatographic analysis, in terms of the odour reduction capacity of these samples and the respective controls without any type of coating, are shown in **Figure 6****.** As can be seen, for the substrate 100% (w/w) CO, there was no considerable increase when incorporating a greater amount of coffee grounds, so it was decided to continue with 25% w/Vₜₒₜₐₗ of coffee grounds.

In an embodiment, for the CO/PES blend fabric, a behavior similar to 100% (w/w) CO fabric is observed, as can be seen in **Figure 7****.** However, the intrinsic reduction capacity of the controls, both with one and with two sides of chitosan, is less than in relation to the 100% (w/w) CO fabric. This result is advantageous, since it makes the effect of grounds more noticeable.

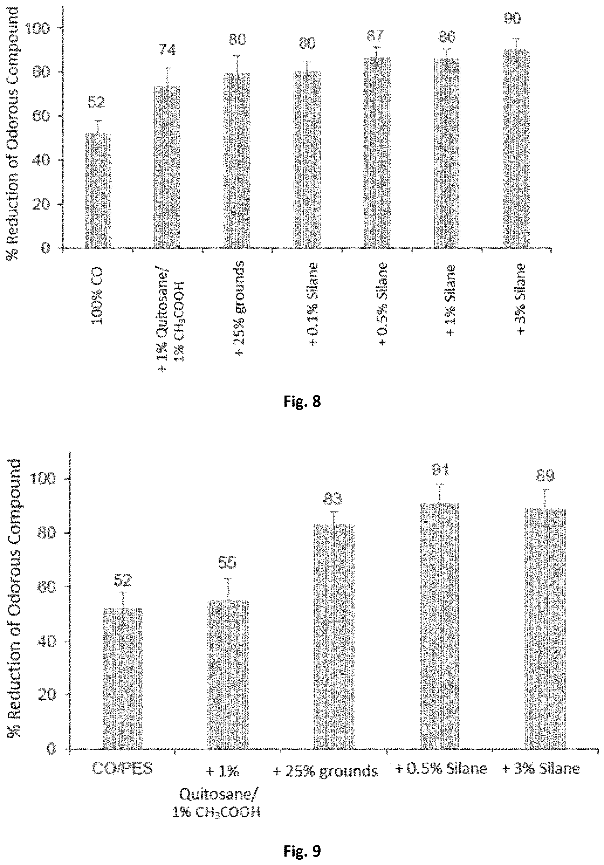

In an embodiment, as shown in **Figure 8** and **Figure 9****,** it appears that silane, in the concentrations tested and for both fabrics, does not have such a marked effect in terms of IVA reduction as in the concentrations previously tested. In addition to reaching a value close to that mentioned in the standard for the reduction of this marker, with these concentrations, an excellent formulation uniformity for spreading over the textile substrate is also obtained. Among the tested concentrations, and taking into account the values obtained for each one, it was decided to proceed with the use of 0.5% (V/V) of silane in future formulations. This is because, regarding the formulation of 0.1 and 1% (V/V) of silane, it reaches a value close to each other and ideal in relation to the standard criterion; and in comparison with the that of 3% it also reaches a close reduction value. In addition, the use of a lower concentration makes the formulation more beneficial for possible direct contact with the skin.

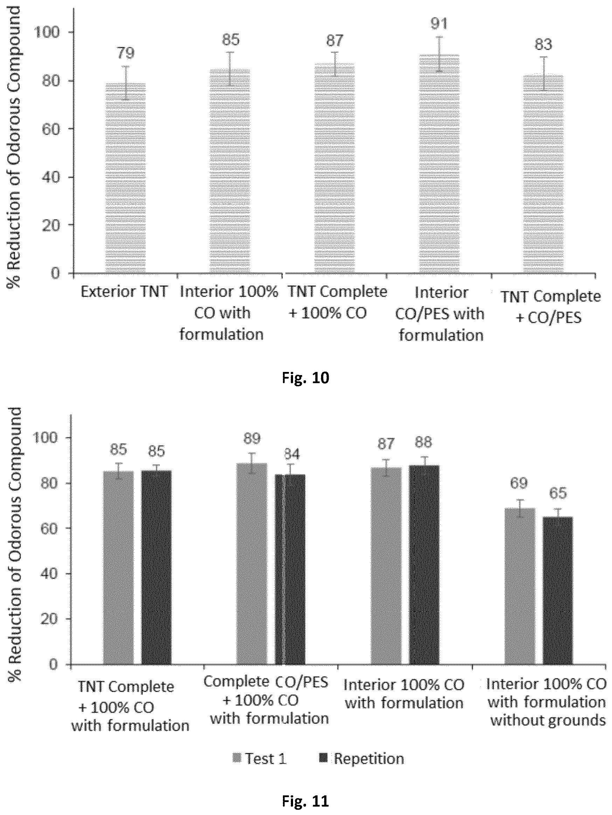

In an embodiment, **Figure 10** shows the results obtained for the dressing developed with the outer layer of TNT and the inner layer of 100% CO or CO/PES. It is possible to verify that, in dressings developed in comparison with the commercial ones, the outer layer has a IVA reduction capacity lower than the inner layer, allowing it to also act in the case of the complete dressing. Both TNT PES with 100% (w/w) CO as well as functionalized CO/PES show significant results in reducing this marker, taking into account the criterion defined in the standard, despite the latter being relatively impaired when complete.

In an embodiment, after each test, the wound dressings of the present embodiment were smelled and it was found that these neutralized/softened the odour of the odorous compound analysed. The same was not seen with the commercial dressings, so these prototypes showed the potential to overcome this problem.

In an embodiment, another way of making the dressings with the spent coffee grounds covered the application of them, loose, inside a kind of bag, which was later covered with an outer layer, using the same combination of fabrics. In the same study, it was also possible to evaluate the performance of this inner layer, with the loose grounds, individually, for direct application on chronic wounds. The amount of loose grounds added was 5 grams in little bags with approximately 50 cm² of area.

In an embodiment, after analysing various parameters in terms of the form and quantity of grounds, the components of the formulation, the fabrics to be used and the most appropriate functionalization technique, the remaining studies followed with only 2 of the developed dressing prototypes. Namely, dressings with the outer layer in CO/PES coupled with the inner layer 100% (w/w) CO with formulation and the outer layer TNT with the inner layer 100% (w/w) CO coated with the formulation under study. The dressing with the outer layer in 100% (w/w) CO and the inner layer of CO/PES coated with the formulation was discarded from the following tests, considering that in terms of medical application it would not be so appropriate.

In an embodiment, **Figure 11** shows that there is consistency in the results obtained, inasmuch as these tests were performed 3 months apart and by different operators. In addition, the preparation of the formulations and the incorporation into the textile substrate 100% CO was also carried out on different days. Therefore, it can be said that the method of incorporating coffee grounds into the textile, the preparation of the dressings and their capacity in terms of the percentage of odour reduction is stable and controlled, not varying significantly.

Another problem of the art was that the commercial dressings, after some time of use, didn't have the capacity to neutralize the bad odour released by exudates of chronic wounds. In this sense, it was decided to evaluate this parameter using two different tests.

In an embodiment, the first trial consisted of assessing the odour retention capacity of the dressings (Actisorb^{®} and the other 2 developed), over a period of 24 hours, allowing to monitor their effectiveness. To this end, 85 µl of IVA were injected in an Erlenmeyer flask, in each dressing, simulating the amount of exudate a leg pressure ulcer releases every half hour [L. F. B. Giraldo, "Extraction and characterization of polysaccharides and phenolic compounds from spent coffee grounds and their incorporation into edible films/coatings for food applications", 2016]. The values were obtained after two hours, consecutive with each injection, until reaching 24 hours of contact with the dressings.

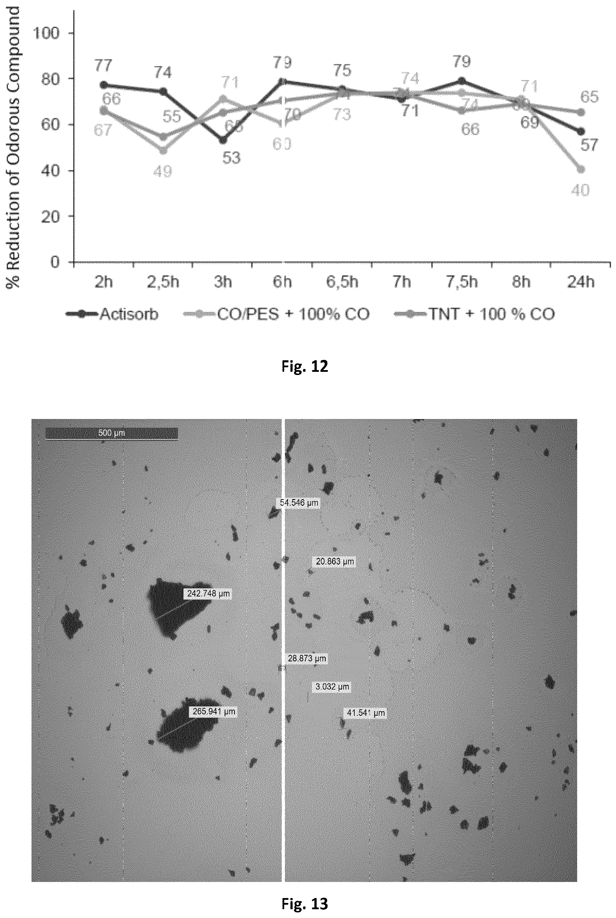

In an embodiment, the results shown in **Figure 12** show that the developed dressings exhibit a behaviour similar to the commercial dressing, in terms of odour reduction, during the 24h of simulation. It should be noted that the dressing exterior in TNT and with the interior of 100% (w/w) CO with formulation has a more linear trend, over time and that after 24 hours the reduction capacity exceeds that of the commercial dressing.

In an embodiment, the odour of the dressings was qualitatively assessed throughout the test - characterized as a weak, medium, or strong odour, in order to determine their neutralization capacity **(Table 1).** In summary, it was noticed that the exterior dressing in TNT and with the interior of 100% CO with formulation only started to present a strong odour of IVA after 24h, while the other two already released a strong odour to the surrounding air after 7h.

**Table 1. Qualifying evaluation of the IVA odour in the 24h test, both for the commercial dressing and for the two developed prototypes.**

| Dressings | Qualification of IVA odour |
|---|---|
| Actisorb^{®} | Strong odour from 7h |
| Exterior TNT + Interior 100% CO with formulation | Strong odour after 24h |
| Exterior CO/PES + Interior 100% CO with formulation | Strong odour from 7h |

From the obtained results, it appears that the developed prototypes have an absorption capacity similar to the Actisorb^{®} commercial dressing, despite being slightly lower. Regarding the Carboflex^{®} commercial dressing, the absorption capacity of the developed dressings is much lower. This result can be explained by the fact that this dressing consists of 4 layers, allowing to absorb a greater amount of solution, compared to the developed prototypes. Anyway, as the developed dressings are structurally similar to Actisorb^{®}, it can be said that in terms of absorption capacity the developed prototypes show satisfactory results. In general terms, dressings with greater absorption capacity are those that have the outside in TNT and an inner layer with grounds in formulation or loose.

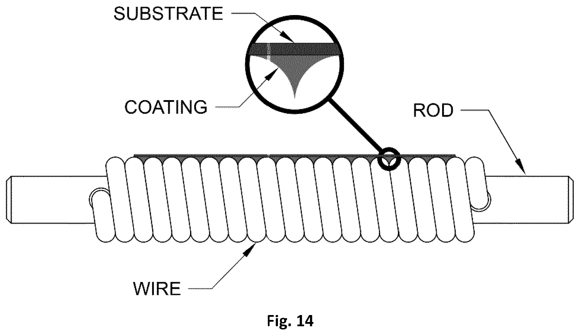

In the present embodiment, figure 14 shows a Meyer bar as a technology for applying coatings, similar to a film applicator.
In this coating process, the formulation is deposited on the substrate and is displaced by means of a dosing rod wrapped in wire (Meyer bar). The Meyer bar allows the desired amount of coating to remain on the substrate and the amount is determined by the diameter of the wire used in the rod, in this case a wire with a diameter of 100 µm was used.

In an embodiment, the wound treatment dressings described in the present disclosure were sterilized by gamma radiation in the Unidade Tecnológica de Radioesterilização of the Instituto Superior Técnico with different doses of radiation (5, 15 and 25 kGy). The evaluation of the microbial load before and after sterilization was carried out at the Centro Tecnológico CITEVE - Tecnologia Têxtil (reports attached).

**Table 2 - Results of the microbiological control of the dressings of the present disclosure by the Portuguese Pharmacopoeia 2.6.12**

| | | **Total No. of viable aerobic germs (CFU/g)** | **Bacteria, (CFU/g)** | **Fungi, (CFU/g)** |
|---|---|---|---|---|
| 1^{st} test | Without sterilization | 2166 | 1770 | 396 |
| 2^{nd} test | Without sterilization | 1632 | 452 | 1180 |
| | 5 KGy | 611 | 608 | 3.33 |
| | 15 KGy | 85 | 73.4 | 11.3 |
| | 25 KGy | 16 | 8.95 | 6.69 |

The term "comprises" or "comprising" when used herein is intended to indicate the presence of stated features, elements, integers, steps, and components, but not to preclude the presence or addition of one or more other features, elements, integers, steps and components, or groups thereof.

The following claims define further embodiments of the present description.

## Claims

1. Wound treatment dressing comprising:
an absorbent layer comprising an odour-inhibiting mixture;
wherein the odour-inhibiting mixture comprises spent coffee grounds, an adhesion agent, a dispersant, a binding agent and a buffer solution;
wherein the granulometry of the spent ground coffee grounds varies between 3-5000 µm;
wherein the odour-inhibiting mixture comprises 0.5 - 5 % (V/V_{inhibiting mixture}) of adhesion agent and 20-25% (W/V_{inhibiting mixture}) of spent coffee grounds.

2. Dressing according to the preceding claim wherein the granulometry of the spent ground coffee grounds varies between 4-4000 µm; more preferably 5-3000 µm.

3. Dressing according to any one of the preceding claims wherein the absorbent layer comprises between 0.05 to 5 g of spent coffee grounds per cm² of absorbent layer; preferably between 0.2 to 2.5 g of spent coffee grounds per cm² of absorbent layer.

4. Dressing according to any one of the preceding claims wherein the dressing further comprises an outer layer comprising a textile substrate, which serves as a support for the absorbent layer carrying the inhibiting mixture.

5. Dressing according to any one of the preceding claims wherein the absorbent layer further comprises superabsorbent polymers.

6. Dressing according to any one of the preceding claims which comprises 2-3% (V/V_{inhibiting} mixture) of adhesion agent; preferably 2 - 2.5% (V/V_{inhibiting} mixture) of adhesion agent.

7. Dressing according to any one of the preceding claims wherein the binding agent is selected from a list comprised of: chitosan, gelatin, pectin, cellulose, cellulose derivatives, glucomannan, acrylic emulsions, styrene-butadiene emulsions, styrene-acrylic emulsions, polyurethane-based dispersions, or combinations thereof.

8. Dressing according to any one of the preceding claims wherein the absorbent layer is coated through a Meyer bar and/or film applicator.

9. Dressing according to the preceding claim wherein the diameter of the wire used on the Meyer bar rod comprises between 100-110 µm.

10. Dressing according to any one of the preceding claims wherein the pH range of the buffer solution is comprised between 6 and 10.

11. Odour-inhibiting mixture composition according to claim 1 for use in wound treatment transdermal dressing.

12. Use of the dressing described in any of the preceding claims 1- 10 as an odour absorber in wounds.

13. Method for producing a dressing according to any one of the preceding claims 1-10 comprising the following steps:
preparing the odour-inhibiting mixture comprising spent coffee grounds and an adhesion agent according to claim 1;
placing the odour-inhibiting mixture in the absorbent layer;
applying the absorbent layer on a textile substrate;
drying in the oven, preferably infrared (IR) at 100 °C, the substrate with the composition;
pressing or laminating the structure in order to obtain a dressing, preferably pressing at 110 °C with a pressure of 0. 6MPa (6 bar) for 1 minute.

## Patentansprüche

1. Wundbehandlungsverband, umfassend:
eine Absorptionsschicht, umfassend eine geruchshemmende Mischung;
wobei die geruchshemmende Mischung Kaffeesatz, ein Haftmittel, ein Dispergiermittel, ein Bindemittel und eine Pufferlösung umfasst;
wobei die Korngröße des Kaffeesatzes zwischen 3-5000 µm variiert;
wobei die geruchshemmende Mischung 0,5-5 % (V/V_{der hemmenden Mischung}) Haftmittel und 20-25 % (W/V_{der} hemmenden Mischung) Kaffeesatz enthält.

2. Verband nach dem vorangehenden Anspruch, wobei die Korngröße des Kaffeesatzes zwischen 4-4000 µm; besonders bevorzugt zwischen 5-3000 µm variiert.

3. Verband nach einem der vorangehenden Ansprüche, wobei die Absorptionsschicht zwischen 0,05 und 5 g Kaffeesatz pro cm² der Absorptionsschicht enthält; bevorzugt zwischen 0,2 und 2,5 g Kaffeesatz pro cm² der Absorptionsschicht.

4. Verband nach einem der vorangehenden Ansprüche, wobei der Verband ferner eine äußere Schicht umfasst, die ein textiles Substrat umfasst, das als Träger für die die hemmende Mischung tragende Absorptionsschicht dient.

5. Verband nach einem der vorangehenden Ansprüche, wobei die Absorptionsschicht ferner superabsorbierende Polymere umfasst.

6. Verband nach einem der vorangehenden Ansprüche, umfassend 2-3 % (V/V_{der} hemmenden Mischung) eines Haftmittels; bevorzugt 2-2,5 % (V/Vder hemmenden Mischung) eines Haftmittels.

7. Verband nach einem der vorangehenden Ansprüche, wobei das Bindemittel ausgewählt ist aus einer Gruppe, bestehend aus: Chitosan, Gelatine, Pektin, Cellulose, Cellulosederivate, Glucomannan, Acrylemulsionen, Styrol-Butadien-Emulsionen, Styrol-Acryl-Emulsionen, Dispersionen auf Polyurethanbasis oder Kombinationen davon.

8. Verband nach einem der vorangehenden Ansprüche, wobei die Absorptionsschicht mittels einer Drahtrakel (Meyer-Bar) und/oder eines Filmapplikators aufgetragen wird.

9. Verband nach dem vorangehenden Anspruch, wobei der Durchmesser des an der Drahtrakel verwendeten Drahtes zwischen 100-110 µm beträgt.

10. Verband nach einem der vorangehenden Ansprüche, wobei der pH-Bereich der Pufferlösung zwischen 6 und 10 liegt.

11. Geruchshemmende Mischungszusammensetzung nach Anspruch 1 zur Verwendung in einem transdermalen Wundbehandlungsverband.

12. Verwendung des in einem der vorangehenden Ansprüche 1-10 beschriebenen Verbandes als Geruchsabsorber bei Wunden.

13. Verfahren zur Herstellung eines Verbands nach einem der vorangehenden Ansprüche 1-10, umfassend die folgenden Schritte:
Herstellen der geruchshemmenden Mischung, umfassend Kaffeesatz und ein Haftmittel nach Anspruch 1;
Einbringen der geruchshemmenden Mischung in die Absorptionsschicht;
Aufbringen der absorbierenden Schicht auf ein textiles Substrat;
Trocknen des Substrats mit der Zusammensetzung in einem Ofen, bevorzugt einem Infrarotofen (IR), bei 100 °C;
Verpressen oder Laminieren der Struktur zur Herstellung eines Verbands, bevorzugt durch Verpressen bei 110 °C bei einem Druck von 0,6 MPa (6 bar) für 1 Minute.

## Revendications

1. Pansement pour le traitement de plaies, comprenant :
une couche absorbante comprenant un mélange inhibiteur d'odeurs ;
dans lequel le mélange inhibiteur d'odeurs comprend du marc de café, un agent d'adhérence, un dispersant, un liant et une solution tampon ;
dans lequel la granulométrie du marc de café varie entre 3 et 5000µm ;
dans lequel le mélange inhibiteur d'odeurs comprend 0,5 - 5% (volume/volume_{mélange inhibiteur}) d'agent d'adhérence et 20 - 25% (poids/volume_{mélange inhibiteur}) de marc de café.

2. Pansement selon la revendication précédente, dans lequel la granulométrie du marc de café varie entre 4 et 4000µm ; plus préférablement entre 5 et 3000µm.

3. Pansement selon l'une quelconque des revendications précédentes dans lequel la couche absorbante comprend entre 0,05 et 5g de marc de café par cm² de couche absorbante ; préférablement entre 0,2 et 2,5g de marc de café par cm² de couche absorbante.

4. Pansement selon l'une quelconque des revendications précédentes dans lequel le pansement comprend également une couche externe comprenant un substrat textile, qui sert de support pour la couche absorbante contenant le mélange inhibiteur.

5. Pansement selon l'une quelconque des revendications précédentes dans lequel la couche absorbante comprend également des polymères superabsorbants.

6. Pansement selon l'une quelconque des revendications précédentes qui comprend 2 - 3% (volume/volume_{mélange inhibiteur}) d'agent d'adhérence ; préférablement 2 - 2,5% (volume/volume_{mélange inhibiteur}) d'agent d'adhérence.

7. Pansement selon l'une quelconque des revendications précédentes dans lequel le liant est choisi parmi une liste composée de : chitosane, gélatine, pectine, cellulose, dérivés de cellulose, glucomannane, émulsions acryliques, émulsions de styrène-butadiène, émulsions styrène-acryliques, dispersions à base de polyuréthane, ou des combinaisons de ceux-ci.

8. Pansement selon l'une quelconque des revendications précédentes dans lequel la couche absorbante est enduite par barre de Meyer et/ou applicateur de film.

9. Pansement selon la revendication précédente dans lequel le diamètre du fil utilisé sur la tige de la barre de Meyer est situé entre 100 et 110µm.

10. Pansement selon l'une quelconque des revendications précédentes dans lequel la plage de pH de la solution tampon est située entre 6 et 10.

11. Composition de mélange inhibiteur d'odeurs selon la revendication 1 destinée à être utilisé dans un pansement transdermique pour le traitement de plaies.

12. Utilisation du pansement décrit dans l'une quelconque des revendications précédentes 1-10 en tant qu'absorbeur d'odeurs dans les plaies.

13. Procédé pour la production d'un pansement selon l'une quelconque des revendications précédentes 1- 10 comprenant les étapes suivantes :
préparer le mélange inhibiteur d'odeurs comprenant le marc de café et un agent d'adhérence selon la revendication 1 ;
placer le mélange inhibiteur d'odeurs dans la couche absorbante ;
appliquer la couche absorbante sur un substrat textile ;
sécher le substrat comprenant la composition au four, préférablement à infrarouge (IR) à 100°C ;
presser ou laminer la structure afin d'obtenir un pansement, préférablement pressant à 110°C à une pression de 0,6 MPa (6 bar) pendant 1 minute.
